# EUROPEAN PATENT APPLICATION

(11) **EP 2 415 476 A1**
(43) Date of publication of application: **08.02.2012**
(21) Application number: 10758913.7
(22) Date of filing: 30.03.2010
(51) Int. Cl.: A61K 36/18, A23L 1/30, A61P 17/02, A61P 17/16, A61P 17/18

(54) **COMPOSITION FOR TREATMENT AND/OR PREVENTION OF DERMATOPATHY**

(30) Priority: 31.03.2009 JP 2009085411
(71) Applicant: Lotte Co., Ltd., Tokyo 160-0023 (JP)
(72) Inventor: HIGUCHI, Hiroaki, Saitama-shi Saitama 336-0027 (JP); KURODA, Reiko, Saitama-shi Saitama 336-0027 (JP); NARISE, Atsushi, Saitama-shi Saitama 336-0027 (JP); SHIMIZU, Katsumasa, Saitama-shi Saitama 336-0027 (JP); OSAWA, Kenji, Saitama-shi Saitama 336-0027 (JP); NOMURA, Yoshihiro, Fuchu-shi Tokyo 183-8509 (JP)
(74) Representative: Blodig, Wolfgang
(86) International application number: PCT/JP2010/056127
(87) International publication number: WO 2010/114149

(57) **Abstract**

To provide a composition for treatment and/or prevention of skin disorder, intensive research has been conducted. As a result, a mangosteen (*Garcinia mangostana L.*) pericarp extract has been found to have a reducing effect on the skin disorder. The present invention provides a composition for treatment and/or prevention of skin disorder, the composition including a mangosteen pericarp extract.

## Description

### TECHNICAL FIELD

The present invention relates to compositions having an extract from a mangosteen pericarp to treat and/or prevent skin disorder.

### BACKGROUND ART

Skin disorder such as creases, lower elasticity, and lower water retention capacity of the skin sometimes causes those having the skin disorder to look older, to have an impaired appearance, or further result in discomfort such as itching and pain. Hence, treatment or prevention of the skin disorder is desired.

While the causes of skin disorders have not been completely clarified, most of them are considered to be caused by reactive oxygen. The reactive oxygen refers to a chemical species having chemically active oxygen, and is very unstable and exhibits strong oxidizing power. Examples of the known reactive oxygen include a superoxide anion radical, a hydroxyl radical, hydrogen peroxide, and singlet oxygen. In the meantime, *in vivo,* there is an antioxidant action which removes reactive oxygen by functions of enzymes such as superoxide dismutase (SOD), catalase, and glutathione peroxidase, as well as vitamin E, vitamin C, β-carotene, uric acid, etc. However, excessive production of the reactive oxygen overwhelms the antioxidant action that the organism body possesses. Such a condition is referred to as an oxidative stress condition. Under the oxidative stress condition, proteins, lipids, sugars, nucleic acid, etc., are oxidized.

In a skin tissue, epidermic keratinocytes are subjected to damage due to the reactive oxygen, and metabolism of the extracellular matrix is disturbed. As a result, this leads to nonuniformity of the thickness of the stratum corneum, dryness due to a decrease in barrier functions associated with the stratum corneum, and further creases. In addition, in the dermis, fibroblasts are damaged by reactive oxygen, which causes a decrease in an amount of collagen in the dermis, promotion of cross-link formation between collagens, and then a flexibility or stretchability of the dermis is decreased. The following reasoning seems to account for details of such mechanisms. Specifically, reactive oxygen affects cellular receptors in an epidermic tissue and their ligands, and causes production of cytokines such as interleukin 1 (IL-1) and tumor necrosis factor-α (TNF-α) from epidermic keratinocytes and fibroblasts in the dermis. In addition, the reactive oxygen affects transcription factors and induces activation of AP-1(activator protein-1) and NF-κβ, and an increase in production of matrix metalloproteases (MMPs). Especially, the activation of MMPs seems to form creases and to facilitate aging.

Ultraviolet light is known as a representative example which induces reactive oxygen in a skin tissue. Ultraviolet light is invisible light having a range of 10 to 400 nm, and is classified into UVA (315 to 400 nm), UVB (280 to 315 nm), and UVC (280 nm or less) according to a difference in effects on a human and an environment. Generally speaking, a kind of ultraviolet light that a human regularly receives is primarily sunlight. The sunlight includes ultraviolet light having each wavelength of UVA, UVB, and UVC. Among them, UVC is absorbed in the ozone layer, and hardly reaches the surface of the earth. However, a part of UVA and UVB reaches the surface of the earth, and causes various changes in the skin. Exposure with ultraviolet light results in production of hydrogen peroxide, a superoxide anion, singlet oxygen, and the like in the skin tissue. Years of repeated exposure with ultraviolet light exert malignant effects on the structure and function of the skin. Such phenomenon is called photoaging. In addition, inflammation caused by ultraviolet irradiation (i.e., sun burn) is known to further induce generation of reactive oxygen in the skin tissue. An influence of ultraviolet light exposure on the dermis is verified to be partially regulated by the epidermis.

A mangosteen is an evergreen tall tree which belongs to *Clusiaceae* (*Guttiferae*) *Garcinia,* and originates from a region proximal to Malay Peninsula. Nowadays mangosteen is introduced to Thailand, India, Sri Lanka, Malaysia, and others, and is cultivated as a fruit tree. Besides the above, mangosteen has been used as a natural medicine in Southeast Asia region through the ages, and has been known to have an anti-inflammatory action and an antimicrobial effect. Thus, mangosteen has been used as an antipyretic or anti-infective drug, and used for treating inflammation and injury of the skin.

In respect to an antioxidant action of mangosteen, following reports have been made. A mangosteen pericarp extract contains various active ingredients. Examples of the active ingredients that are known to be abundantly contained include xanthone that possesses a very potent antioxidant action, as well as catechin, polyphenol, polysaccharides, minerals, and vitamins. Among them, research on xanthone, one of polyphenol, has been advanced, and various xanthones have been reported (Journal of Agricultural and food chemistry 54: 2077-2082, 2006). Examples of a representative unique xanthone that is contained in a mangosteen pericarp include α-mangostin and γ-mangostin, and they have been demonstrated to have a reducing effect on oxidation of lipids. In particular, it has been revealed that γ-mangostin has a stronger oxidation-reducing effect than α-tocopherol and BHA, and possesses a radical-removing action equivalent to that of α-tocopherol (YAKUGAKU ZASSHI 114(2): 129-133, 1994). On the other hand, α-mangostin and γ-mangostin have been also known to have an antihistaminic effect or an antiserotonergic effect (Japanese Patent No. 3968405).

In the meanwhile, regarding mangosteen's effect on improvement, treatment, and the like of the skin or cutis, following reports have been made. Japanese Patent Application Laid-Open No. 2007-31287 discloses cosmetics containing Pandanus fruit components, and also discloses that a mangosteen extract is formulated in the specification. Japanese Patent Application Laid-Open No. 2002-47125 discloses an agent for inhibiting sebaceous secretion, the agent comprising a matrix metalloprotease inhibitor, and a mangosteen extract includes the inhibitor. Also, Japanese Patent Application Laid-Open No. H09-87155 discloses an ultraviolet absorber comprising a mangosteen as an active ingredient. In addition, Japanese Patent Application No. 2008-134246 reports that a mangosteen extract effects treatment or prevention of atopic dermatitis by its oral ingestion. However, in this report, the subjects are limited to atopic dermatitis. That is, any of these reports fails to disclose that the mangosteen extract can be used as a composition for treatment and/or prevention of the skin disorder without including other active ingredients.

In addition, varieties of safety studies have been conducted in respect to a mangosteen pericarp extract. Paragraph [0015] of Japanese Patent Application Laid-Open No. H05-17365 recites that an acute toxicity study has been conducted in mice by employing oral administration, and indicates that administration of 10 g/kg of mangosteen pericarp extract is recognized to have no fatal cases. Further, a safety study disclosed in paragraph [0047] of Japanese Patent Application Laid-Open No. H04-244004 demonstrates that an irritation study in which a mangosteen pericarp extract is applied to human skin has been conducted, and proves to have extremely low irritation and high safety.

In contrast, other than the mangosteen, for example, polyphenol in green tea and proanthocyanidin in grape seeds are known as an ingredient for lowering or removing actions of reactive oxygen. The polyphenol in green tea has been reported to have a reducing effect on oxidative damage and MMP expression due to UV in the skin of hairless mice (Journal of investigative Dermatology 122:1480-1487, 2004). It has been reported that proanthocyanidin in grape seeds reduces oxidative stress due to UVB, and inhibits activation of MAPK or NF-κβ (Molecular Cancer Therapeutics 6(3): 995-1005, 2007).

### DISCLOSURE OF THE INVENTION

Any of the above literatures fails to disclose that a mangosteen extract possesses an effect on treatment and/or prevention of skin disorder. It is an object of the present invention to provide a composition for treatment and/or prevention of skin disorder, the composition comprising an extract which is extracted from a mangosteen (*Garcinia mangostana L.*) pericarp by using a polar solvent.

### Means for Solving the Problems

The inventors of the present application have conducted intensive research so as to provide a composition for treatment and/or prevention of skin disorder, the composition comprising an extract which is extracted from a mangosteen (*Garcinia mangostana L.*) pericarp by using a polar solvent. As a result, the inventors have found that an extract which is extracted from a mangosteen pericarp by using a polar solvent can be effective in treatment and/or prevention of skin disorder, and have completed the present invention.

That is, an aspect of the present invention provides a composition for treatment and/or prevention of skin disorder, the composition comprising an extract which is extracted from a mangosteen (*Garcinia mangostana L.*) pericarp by using a polar solvent.

An aspect of the present invention provides the composition for treatment and/or prevention of skin disorder, wherein the skin disorder is caused by reactive oxygen.

An aspect of the present invention provides the composition for treatment and/or prevention of skin disorder, wherein the skin disorder is caused by ultraviolet irradiation.

Further, an aspect of the present invention provides a food comprising the composition for treatment and/or prevention of skin disorder according to the previous aspects.

In an aspect of the present invention, a mangosteen pericarp can employ those obtained from a mangosteen fruit (a fresh or dried product). The mangosteen pericarp can be used as it is. However, when an improvement in extraction efficiency is taken into consideration, it is preferable to be extracted after homogenized or powdered. Also, before the extraction, the mangosteen pericarp can be degreased by using a non-polar solvent.

Extraction according to the present invention is carried out by using at least one solvent (a polar solvent) selected from the group consisting of methanol, ethanol, n-propanol, 2-propanol, n-butanol, acetone, ethyl acetate, and water. Two or more kinds of the solvent can be combined to be carried out. In addition, when it is taken into consideration that the solvent is used in an agent for oral administration or beverage and food, ethanol or a combination of water and ethanol is preferable to be used as an extracting solvent from a viewpoint of safety. The temperature of extraction is not specifically defined. However, in view of extraction efficiency, the temperature is preferably within a range between room temperature and a boiling point temperature of the solvent. An extraction period varies depending on types of the solvent, conditions of pericarp (e.g., a fresh or dried product, a homogenate or powder), and the temperature of extraction, but is preferably within a range between 0.5 and 24 hours.

As to an extract, an extracting solvent may be enriched or removed by an evaporator, etc. as needed. Also, the extract can be used by purifying it by solvent fractionation or chromatography as needed.

The term "skin" herein means the same as the "cutis", and refers to a layer of the body surface of animals. The skin includes epidermis, dermis, and subcutaneous tissue. The term "skin disorder" herein refers to a condition having at least one condition selected from the group consisting of skin inflammation, moisture reduction, lower flexibility, and occurrence of creases.

The term "reactive oxygen" herein refers to a chemical species in which oxygen becomes chemically active, and those which exhibit marked instability and potent oxidizing power. Examples of the reactive oxygen can include a superoxide anion radical, a hydroxyl radical, hydrogen peroxide, singlet oxygen, nitric oxide, nitrogen dioxide, ozone, and lipid peroxide.

The term "ultraviolet light" herein refers to light having a wavelength between 10 and 400 nm.

A method for administering a composition for treatment and/or prevention of skin disorder according to the present invention is not limited, but the composition is preferably orally ingested. Accordingly, a composition for treatment and/or prevention of skin disorder according to the present invention can be added to a soft drink, a snack, a frozen dessert, a dairy product, alcoholic liquor, and food such as meat.

An amount of administration of a mangosteen pericarp extract as a composition for treatment and/or prevention of skin disorder varies depending on a method for administration and necessary treatment, and is not uniformly defined. However, when orally ingested, an amount of the extract is kept between 60 and 250 mg per kg body weight of an animal. As to a human, the amount resides between 0.3 mg and 300 mg/kg body weight/day, and more preferably between 0.5 mg and 200 mg/kg body weight/day.

As to a composition for treatment and/or prevention of skin disorder according to the present invention, a formulation amount can be defined so as to satisfy the above effective amount by daily regular intake as food. A daily amount of the ingestion can be divided into several portions.

### Advantages of the Invention

An aspect of the present invention has provided a composition for treatment and/or prevention of skin disorder, the composition comprising a mangosteen pericarp extract.

A mangosteen is said to be "the Queen of fruits", and the fruit thereof is supplied for edible use. The mangosteen is famous food stuff holding a good image. Accordingly, a composition for treatment and/or prevention of skin disorder is readily acceptable for consumers, the composition comprising a mangosteen pericarp extract. Then, the composition is preferably employed as a composition which is added to food. Further, the present invention uses an extract of a pericarp which is usually wasted among mangosteen parts. Because of this, source materials are available at a low price, and are also desirable from a viewpoint of environmental protection.

In addition, an extract of the present invention can be extracted from a mangosteen by using a polar solvent, and allows the above effect to be achieved without further purification.

### BRIEF DESCRIPTION OF THE DRAWINGS

FIG. 1 shows a schedule of mangosteen pericarp extract-mixed-diet administration experiments.
FIG. 2 shows spectral distribution of a UVB lamp.
FIG. 3 is a graph illustrating various parameters of Cutometer SEM 575.
FIG. 4 indicates body weight. FIG. 4A is a graph showing transition of body weight. FIG. 4B presents all the data.
FIG. 5 indicates an amount of food ingested. FIG. 5A is a graph showing transition of an amount of food ingested. FIG. 5B presents data regarding an amount of food ingested.
FIG. 6 indicates skin moisture content. FIG. 6A presents changes over time in moisture content and data of the humidity at measurements. FIG. 6B shows moisture content at week 8.
FIG. 7 indicates skin elasticity. FIG. 7A shows skin elasticity at the time of grouping. FIG. 7B shows skin elasticity at the time of dissection.
FIG. 8 is photographs showing appearance of dorsal skin. FIG. 8A is a photograph showing appearance of hairless mouse No. 3 of the control (-) group. FIG. 8B is a photograph showing appearance of hairless mouse No. 26 of the control (+) group. FIG. 8C is a photograph showing appearance of hairless mouse No. 36 of the 0.15% (+) group.

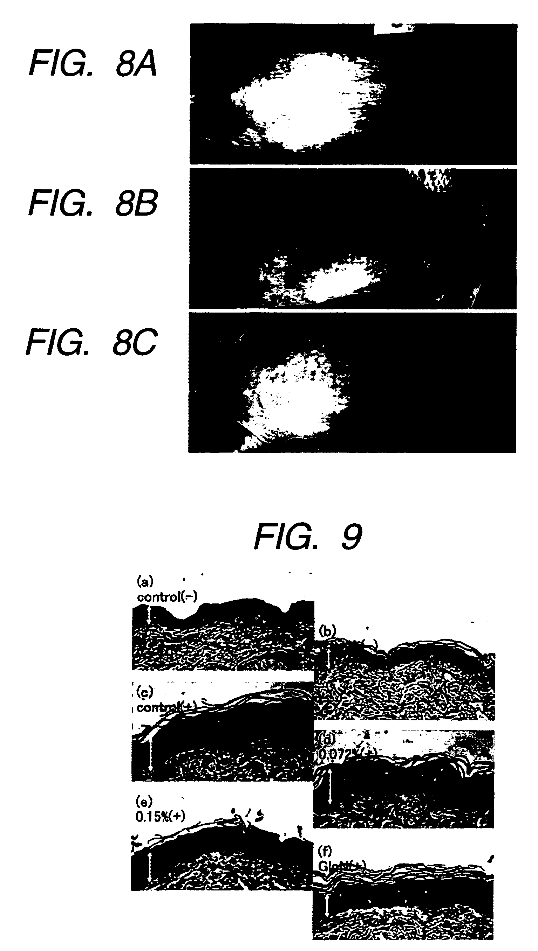

FIG. 9 is photographs showing HE-stained specimens of hairless mice of a photoaging model. FIG. 9(a) shows a photograph of a specimen of hairless mouse No. 9 of the control (-) group. FIG. 9(b) shows a photograph of a specimen of hairless mouse No. 11 of the 0.072% (-) group. FIG. 9(c) shows a photograph of a specimen of hairless mouse No. 25 of the control (+) group. FIG. 9(d) shows a photograph of a specimen of hairless mouse No. 29 of the 0.072% (+) group. FIG. 9(e) shows a photograph of a specimen of hairless mouse No. 41 of the 0.15% (+) group. FIG. 9(f) shows a photograph of a specimen of hairless mouse No. 50 of the GlcN (+) group.
FIG. 10 is a graph indicating the thickness of the epidermis by calculating using pathologic HE-stained specimens.
FIG. 11 is a picture showing a result of SDS-PAGE of a skin extract.
FIG. 12 shows a result of Western blotting for detecting type I collagen. FIG. 12A is a picture showing a result of Western blotting of a skin extract. FIG. 12B is a table indicating band intensity determined by densitometry analysis.
FIG. 13A is a picture indicating identification of GAG by cellulose acetate membrane electrophoresis. FIG. 13B is a table indicating band intensity determined by densitometry analysis.
FIG. 14 is a graph showing results of determining lipid peroxide by a TBA method.
FIG. 15 is a picture showing a result of SDS-PAGE of a skin extract.
FIG. 16A is a picture showing a result of detecting carbonyl proteins by Western blotting. FIG. 16B is a table indicating band intensity determined by densitometry analysis.
FIG. 17 is a graph showing results of measuring lipid peroxide in plasma.
FIG. 18 is a diagram showing a schedule of experiments for oral forced administration of a mangosteen pericarp extract.
FIG. 19 is a diagram illustrating steps of extracting soluble components from a skin tissue.
FIG. 20 shows moisture content. FIG. 20A is a graph showing its transition. FIG. 20B presents all the data.
FIG. 21A is a table indicating skin elasticity.
FIG. 21B is a graph indicating R0: stretchability.
FIG. 21C is a graph indicating R1: force which puts the skin back into an original state.
FIG. 21D is a graph indicating R2: total elasticity.
FIG. 21E is a graph indicating R3.
FIG. 21F is a graph indicating R4.
FIG. 21G is a graph indicating R5.
FIG. 21H is a graph indicating R6.
FIG. 21I is a graph indicating R7.
FIG. 21J is a graph indicating R8.
FIG. 22 is photographs to illustrate how to interpret specimens from a skin tissue. FIG. 22(a) is a photograph representing a non-UV-irradiated specimen. FIG. 22(b) is photographs representing a UV-irradiated specimen.
FIG. 23 is photographs showing HE-stained pathological specimens of the dorsal skin.
FIG. 24 is a graph indicating the thickness of the epidermis determined using HE-stained specimens.
FIG. 25 shows results of SDS-PAGE and Western blotting for detecting type I collagen. FIG. 25A is a picture showing a result of SDS-PAGE of a skin extract. FIG. 25B is a picture showing a result of Western blotting. FIG. 25C is a table indicating relative band intensity determined by densitometry analysis.
FIG. 26 shows results of SDS-PAGE and Western blotting for detecting decorin. FIG. 26A is a picture showing a result of SDS-PAGE of a skin extract. FIG. 26B is a picture showing a result of Western blotting. FIG. 26C is a table indicating relative band intensity determined by densitometry analysis.

### BEST MODES FOR CARRYING OUT THE INVENTION

Hereinafter, the present invention is illustrated by referring to Examples. However, the scope of the present invention is not limited to the following Examples.

### Example 1

### Preparation of a mangosteen pericarp extract

A mangosteen pericarp extract is obtained as follows. Specifically, 100 g of an undried pericarp of a mangosteen was ground, and extracted at 80°C in 1 l of 70% ethanol for 1 hour while stirring. This solution was filtered, and the filtrate was dried under reduced pressure by using an evaporator to yield 27.4 g of an extract.

### Example 2

### Examination of effects of improving skin disorder by administration of a mixed diet made by blending a mangosteen pericarp extract

### Experimental Materials and Experimental Methods

### 1. Animals used and their rearing conditions

After one week of acclimatization, 54 six-week-old male Hos: HR-1 hairless mice were used in experiments. The below-described diet was used, and the mice were reared under conditions at 25°C while keeping a free access to food and water. In addition, during measurements of the skin moisture content, a humidity meter was used to determine humidity in an animal room. All experiments were conducted under approval (No. 19-76) of Animal Care and Use Committee of Tokyo University of Agriculture and Technology.

### 2. Method for preparing an administration sample

Preparation of an administration diet was ordered to Oriental Yeast Co., Ltd. First, 0.072% mangosteen pericarp extract-mixed diet and 0.15% mangosteen pericarp extract-mixed diet were used. In addition, 0.24% glucosamine-mixed diet was used as a positive control, and CRF-1 diet was used as a negative control. A daily amount of mangosteen pericarp extract ingested was set to 120 mg/kg body weight for the 0.072% group, and set to 250 mg/kg body weight for the 0.15% group as a rough guideline.

### 3. Rearing schedule

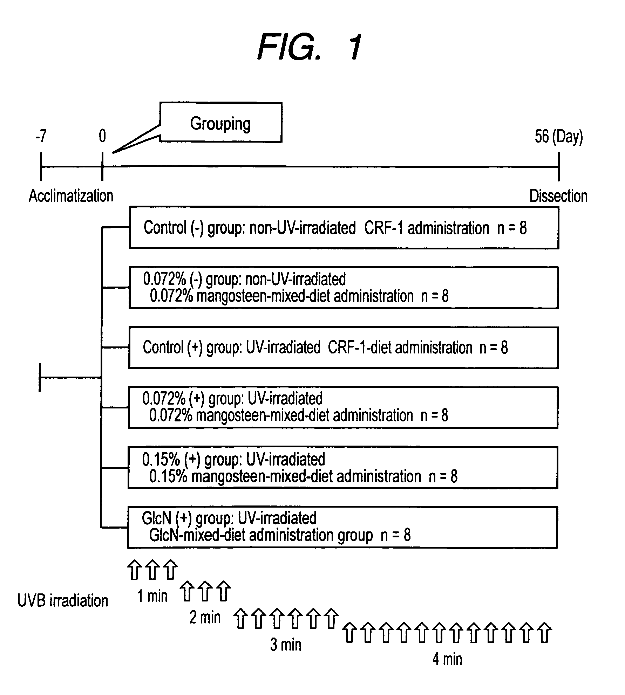

A study schedule is illustrated in FIG. 1. A study period was set to 8 weeks, and the study was carried out from October to December in 2007. After 1 week of acclimatization, by paying attention so as not to have a bias based on skin moisture content and viscoelasticity, animals were divided into six groups including a non-UV-irradiated CRF-1 administration group (control (-) group), a non-UV-irradiated 0.072% mangosteen pericarp extract-mixed-diet administration group (0.072% (-) group), a UV-irradiated CRF-1 administration group (control (+) group), a UV-irradiated 0.072% mangosteen pericarp extract-mixed-diet administration group (0.072% (+) group), a UV-irradiated 0.15% mangosteen pericarp extract-mixed-diet administration group (0.15% (+) group), and a UV-irradiated 0.24% glucosamine (GlcN)-mixed-diet administration group (GlcN group). After the grouping, UV-irradiated groups were irradiated with UV light three times a week. Specifically, for the first week, 1 minute of UV irradiation was carried out. For the second week, 2 minutes of UV irradiation, and for the third and fourth week, 3 minutes of UV irradiation were conducted. From the fourth week to the time of dissection completed, 4 minutes of UV irradiation were carried out. The total irradiation amount was 1.35 J. In addition, moisture content and an amount of food ingested were measured twice a week, and body weight was measured once a week.

### 4. As to UV irradiation

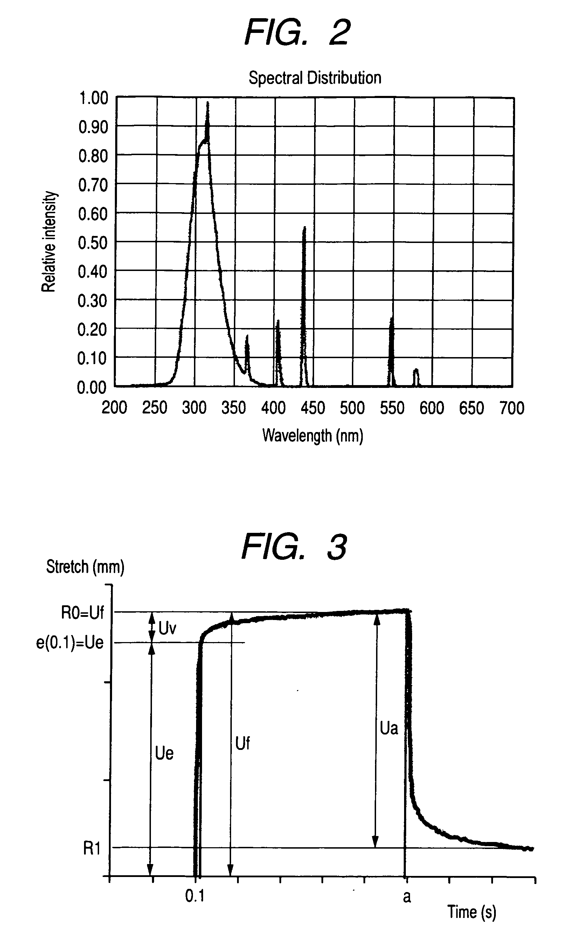

A UVB lump GL20SE (SANKYO DENKI) was used to carry out UV irradiation having irradiation intensity of 0.3 mW/cm². Spectral distribution of a UVB lamp is shown in FIG. 2, and the lamp emits light having a wavelength range of 280 nm or more. There is a peak wavelength of 280 nm. Irradiation intensity was adjusted by using a digital ultraviolet light intensity meter, UV-340 (AS ONE Corporation). During irradiation, mice were left for one and a half hour in an individualized cage having a size of 9 cm × 5 cm × 4 cm. In order to decrease a difference in irradiation intensity for respective cages, the mice were irradiated while subjected to a rotation in every occasion.

### 5. Determination of skin moisture content

Skin moisture content was measured using CORNEOMETER® CM825 (manufactured by COURAGE+KHAZAKA electronic GMBH) twice a week, the measurement being carried out by contacting a probe onto a lumbar region of a mouse prior to UVB irradiation. The measurements were conducted five times, and the average of them was designated as a measured value.

### 6. Skin viscoelasticity measurement

Measurements were carried out by using CUTOMETERⓇ SEM575 (manufactured by KHAZAKA electronic GMBH) at the time of grouping and the time of dissection.

This machine determines the height of the skin which is sucked into a vacuum inlet of a probe by using a photosensor capable of measuring a unit of 1/100 mm. By the measurements, 10 parameters can be obtained as the following r0 to r9. FIG. 3 indicates the definitions of the respective values.
r0 = e(a): a maximum value of amplitude of the first waveform (Uf)
r1 = e(a + b): a minimum value of amplitude of the first waveform, an ability of putting the skin back in the original state (reformation capability)
r2 = (e(a) - e (a+ b))/e(a): (Ua/Uf)
r3 = e((r × a) + ((r-1) × b)): a difference between the maximal amplitude and the reformation capability (a total elasticity)
r4 = e((a + b) × r)
r5 = (e(a) - e(a + 0.1))/e(0.1): a total elasticity of the skin without viscous deformation (Ur/Ue)
r6 = (e(a) - e(0.1))/e(0.1): a proportion of viscosity to elastic expansion (Uv/Ue)
r7 = (e(a) - e(a + 0.1)/e(0.1): a value comparing an elastic portion with a complete waveform (100% elasticity) (Ur/Uf)
r8 = e(b): r8 indicates force of putting the skin back into the original state after release of negative pressure.
r9 = r3 - r0: the skin height caused by vacuum at the cursor point

In this test, a probe with a vacuum inlet having a diameter of 2 mm was used. After 10 seconds of vacuum by using a negative pressure of 300 mbar and subsequent rapid release of the negative pressure, a measurement was carried out by subjecting to 10 seconds of suction removal. The measurements were conducted in triplicate, and the average of them was designated as a measured value.

### 7. Photography of the dorsal skin

In order to observe dorsal skin conditions of mice, pictures were taken by using a digital camera. The dorsal region was photographed twice at the time of grouping and the time prior to dissection after gas anesthesia treatment using isoflurane for mice.

### 8. Method for sampling a skin tissue

Mice were dissected at day 57 of rearing. After blood drawing, a skin tissue was collected. After a skin sample used for pathological analysis was collected using an 8-mm-biopsy punch, a whole dorsal region of the skin was sampled, and a subcutaneous tissue was removed by using a blunt part of a sickle scalpel. The skin tissue was subjected to freezing and crushing in liquid nitrogen by using a JFC-300-type freeze crusher (YOSHIDA SEISAKUSHO Co., Ltd.), and stored at -80°C.

### 9. Histological analysis

The dorsal skin as sampled above was interposed between filter papers to be flattened, and was placed in a 4-cm dish. Then, a few drops of 10 N MildformⓇ (manufactured by Wako Pure Chemical Industries, Ltd.) were dropped thereon to fix the sample. After that, according to a standard protocol, the sample was paraffin-embedded, dissected, sectioned, HE-stained, and prepared for a pathological tissue specimen. The specimen preparation was ordered to Sapporo General Pathology Laboratory.

The entire visual field of these specimens was observed, and a representative field (around the center field of the section and the field without undulation in the tissue) of each individual was photographed. In addition, the thickness of the epidermis was measured for 10 locations of each specimen by using the HE specimens, and the epidermic thickness was determined by calculating the average of them.

### 10. Method for extracting proteins from the skin tissue

A frozen and crushed skin was degreased using methanol for 24 hours. Then, an appropriate amount of PBS (-) containing protease inhibitors was added, and the sample was stirred with a rotator and washed twice to remove serum components, etc. After that, 10 volumes per wet weight of the skin tissue of an extracting buffer (4M GuHCl/50 mM Tris-HCl/0.1 M NaCl/5 mM benzamidine hydrochloride/10 mM EDTA-2Na/0.1 M aminohexanoic acid (pH 7.4)) were added to the sample, and the sample was extracted at 4°C for 72 hours while stirring. Following that, the sample was centrifuged at 4000 rpm for 30 minutes, and the supernatant was subjected to dialysis with RO water. After the dialysis, the sample was lyophilized. This sample was used for detection of collagen.

In addition, two volumes per skin wet weight of another protein-extracting solution (50 mM Tris-HC1/1% Trion X-100/75 mM NaCl/10 mM EDTA-2Na/5 mM benzamidine hydrochloride/0.1 M aminohexanoic acid) was added to the sample, and the sample was extracted. After the extraction, the sample was centrifuged at 4000 rpm for 30 minutes, and the supernatant was filtered using a filter and was subjected to detection of carbonyl proteins.

### 11. Detection by SDS-PAGE and Western blotting

SDS-PAGE was conducted according to a method of Laemmli et *a1.* The lyophilized sample was assayed for proteins according to a Bradford protocol. The protein concentrations for the respective samples were balanced. After 5 minutes of heating at 100°C, the samples were rapidly cooled to prepare samples for electrophoresis. Following electrophoresis, CBB staining was carried out. To detect collagen, 6% acrylamide gel was used. To detect decorin (Example 3), 7.5% acrylamide gel was used to perform electrophoresis. After the electrophoresis, the gel and PVDF membrane were equilibrated with a blotting buffer (25 mM Tris-HCl/190 mM Glycine/0.04% SDS/20% Methanol) for 30 minutes. An ice-cooled wet-type blotting apparatus (manufactured by Bio-Rad) was used, and electrified at 0.05 A for 18 hours. Then, proteins in the gel were transferred onto the membrane. After the transfer, the PVDF membrane was shaken in the blocking solution for 1 hour at room temperature. To detect collagen, the blocking solution employed 5% skim milk/TBS-Tween. To detect decorin, chondroitinase ABC (manufactured by SEIKAGAKU CORPORATION) was added to the 5% skim milk/TBS-Tween to have a proportion of 0.01 U/ml. Then, the membrane was shaken in a primary antibody-containing solution (primary antibody:5% skim milk/TBS-Tween = 1:1000) for 1 hour at room temperature. Next, the membrane was washed with TBS-Tween four times for 5 minutes, and was shaken at room temperature in a secondary antibody-containing solution for 1 hour in a similar manner and was then washed. To detect the antigen, a chemiluminescence method utilizing an HRP reaction was employed. ECL kit (manufactured by Amersham-Pharmacia Biotech, Inc.) was used as a chemical reaction reagent. The membrane was exposed onto a FUJI MEDICAL X-ray film (manufactured by Fuji Film, Inc.), and the film was developed. The developed film was subjected to comparison of the band intensity determined using an image-analyzing software, Scion Image (manufactured by Scion Corporation).

Of note is that detection of collagen used an anti-type-I collagen (derived from porcine skin) rabbit-antiserum as a primary antibody and an HRP-labeled anti-rabbit IgG antibody as a secondary antibody. In addition, detection of decorin used an anti-decorin core protein rabbit-antiserum as a primary antibody and an HRP-labeled anti-rabbit IgG antibody as a secondary antibody.

### 12. Method for extracting glycosaminoglycan (GAG) from a skin tissue

The frozen and crushed skin was degreased at 4°C over night by using ethanol. Next, 30 volumes per wet weight of 0.5 M NaOH was added to the sample. Then, the sample was reacted at 4°C for 20 hours while rotating on a rotator, and a β-elimination reaction occurred to release GAG from proteins. After that, a half volume per NaOH added of 1 M HCl was added to carry out a neutralization reaction. A pH test paper was used to examine whether or not the sample had been neutralized. Here, 1.5 volumes per NaOH of 2 x conc. Actinase buffer was added, and the sample was heat-denatured at 100°C for 10 minutes. When the temperature of the solution returned to 50°C, a piece of thymol was added as a preservative, and Actinase was added to have a concentration of 1% (w/v) per NaOH as originally added. A degradation reaction was carried out for 2 days while using a shaker at 50°C. At this occasion, the same amount of Actinase as originally added was added again 24 hours after the initiation of the reaction. After the reaction was completed, trichloroacetic acid was added to have a final concentration of 10%, and the sample was left at 4°C for 1 hour to remove proteins. Following that, the sample was centrifuged at 0°C and 9000 g for 15 minutes. Then, the supernatant was filtered with a filter by using DISMICⓇ (manufactured by ADVANTEC TOYO, Inc.) and was subjected to dialysis. The post-dialysis solution was lyophilized, and the lyophilized sample was dissolved in Milli-Q water to be used for cellulose acetate membrane electrophoresis.

### 13. Analysis of GAG by cellulose acetate membrane electrophoresis

In order to identify compositions of glycosaminoglycan in the skin of a hairless mouse, cellulose acetate membrane electrophoresis was carried out according to a method of Hata et *a1.* GAG as prepared using the above method was dissolved in 50 µl of Milli-Q water per 100 mg of wet weight of the extracted skin to prepare samples used for electrophoresis. Next, 0.5 µl of sample was spotted onto a cellulose acetate membrane. Electrophoresis was conducted using constant current of 1 mA per cm of the membrane width in use of 0.1 M pyridine/0.47 M performic acid buffer. As to standards, hyaluronic acid (HA), dermatan sulfate (DS), chondroitin sulfate (CS) were each dissolved to have a concentration of 1 mg/ml, and 50 µl of each was mixed to be used. After the electrophoresis was completed, the membrane was stained with Alcian blue staining solution (0.5% Alcian blue/25% ethanol/10% acetic acid), and subsequently destained with 10% acetic acid.

The resulting spots were analyzed by using an image-analyzing software, Scion Image (Scion Corporation).

### 14. Method for determining lipid peroxide in the skin by using a TBA method

A TBA method has been used as a method for comprehensively determining almost all components that have been generated by lipid peroxidation such as lipid peroxide, malondialdehyde and other aldehyde, and reaction products of aldehyde with proteins or the like. The method determines the degree of peroxidation of lipids by quantifying red pigment that is generated during a reaction of thiobarbituric acid (TBA) with a thiobarbituric acid reactive substance (TBARS) that has been released from the sample.

The degree of peroxidation of lipids in the skin was measured according to a method of Ohkawa et *a1.* First, to the frozen and crushed skin was added 1.15% KCl aqueous solution to have a concentration of 15% (w/v) of wet weight, and stirred. To 50 mg of the tissue homogenate were added in the order of 100 µl of 8.1% SDS solution, 0.75 ml of an acetic acid buffer, 25 µl of 0.8% BHT-acetic acid solution (an antioxidant), 0.75 ml of 0.8% TBA aqueous solution, and 350 µl of 5 mM FeCl₃ while strongly stirring. The mixture was kept at 5°C for 60 minutes, and heated in a boiling water bath for 60 minutes. After cooling, 0.5 ml of Milli-Q water and 2.5 ml of a butanol-pyridine-mixed solution (15:1, v/v) were added to be strongly shaken, and the mixture was centrifuged at 3000 rpm for 20 minutes. Absorbance at 532 nm was measured for the supernatant solution, and an amount of TBARS was calculated from a standard curve. At that occasion, as the standard for a calibration curve, 1,1,3,3-tetraethoxypropane (biochemistry grade, Wako Pure Chemical Co., Inc.) was employed. Instead of the frozen and crushed skin, an equivalent amount of a KCl aqueous solution was added to prepare a blank.

### 15. Method for determining oxidized proteins in a skin tissue

Carbonyl proteins are one of oxidized proteins and constitute a marker for oxidative stress. The carbonyl proteins were examined by using Oxyblot™ Protein Oxidation Detection Kit (manufactured by CHEMICON® Inc.). The specific method is described below.

Samples of each group were assayed for proteins by using a BCA method, and the protein concentration of the sample was adjusted to be 1.1 mg/ml. To 5 µl of the protein sample was added 5 µl of 12% SDS. To the resulting sample was added 10 µl of a 2,4-dihydrophenylhydrazone (DNPH) solution, and the mixture was incubated at room temperature for 15 minutes to introduce DNPH into a carbonyl group. After that, 7.5 µl of a neutralizing solution was added to terminate the reaction. DNPH-derivatized samples were subjected to SDS-PAGE using 10% gel to separate proteins. Then, blotting onto a PVDF membrane was carried out using a blotting apparatus. The post-transfer membrane was blocked by soaking into a blocking buffer (5% skim milk/TBS-Tween) under ordinary temperature for 1 hour, and then reacted with a primary antibody under ordinary temperature for 1 hour. After the reaction, the membrane was washed with TBS-Tween four times for 5 minutes, and reacted with a secondary antibody under ordinary temperature for 1 hour. After the reaction, the membrane was washed again four times for 5 minutes. To detect the antigen, a chemiluminescence method utilizing an HRP reaction was employed. ECL kit (Amersham-Pharmacia Biotech, Inc.) was used as a chemical reaction reagent. The membrane was exposed onto a FUJI MEDICAL X-ray film (manufactured by Fuji Film, Inc.), and the film was then developed. The developed film was subjected to comparison of the band intensity determined using an image-analyzing software, Scion Image (Scion Corporation).

### 16. Determination of lipid peroxide (LPO) in plasma

The determination was ordered to NIKKEN SEIL Co., Ltd.

### 17. Statistical processing

Every parameter was examined whether or not there is normality and/or homoscedasticity. Then, if the parameter has normal and homoscedastic distribution, Tukey-kramer test was conducted. Otherwise, Scheffe's F-test was carried out.

### Results

### 1. Basic data

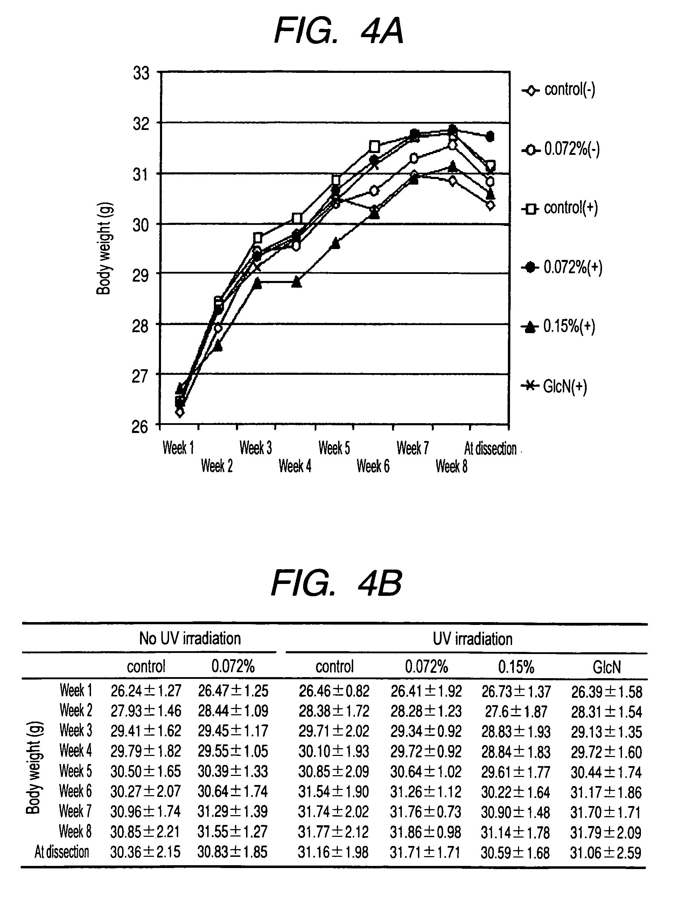

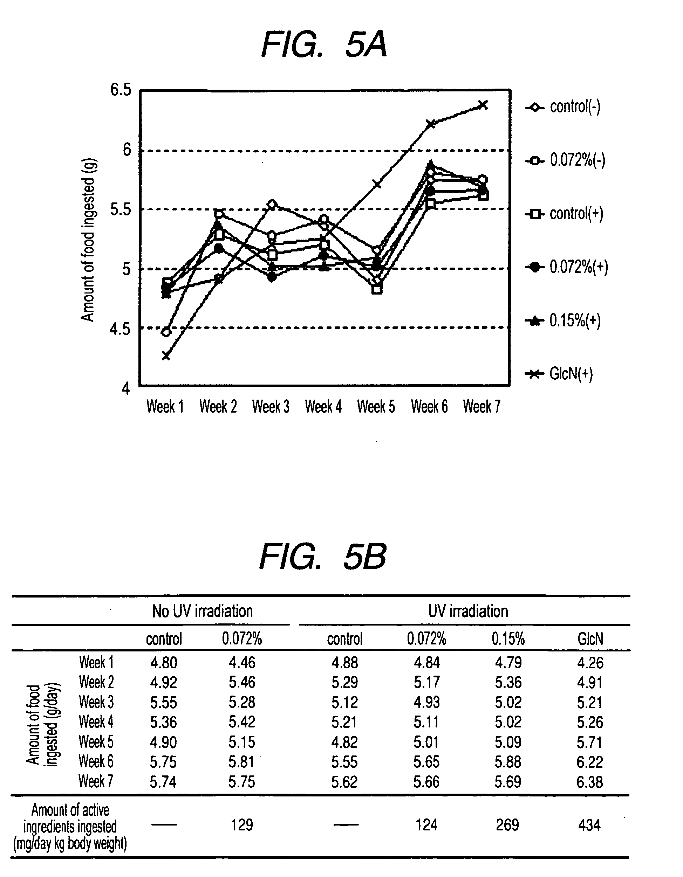

Body weight and an amount of food ingested were determined in order to investigate whether or not there were effects on UV-irradiated and mixed-diet-administered mice. The body weight was measured once a week. The amount of food ingested was measured for each cage twice a week. FIG. 4 shows results of measuring the respective body weight, and FIG. 5 shows results of measuring the respective amount of food ingested. A difference in body weight was not observed among the respective groups. In addition, as to the amount of food ingested, after week 4, the GlcN (+) group tended to have an increased amount of food ingested compared to other groups. However, the increase in the amount of food ingested did not affect its body weight.

The average of the ingested amount of active ingredient for the respective groups was determined by measurements of the amount of food ingested. The average for the 0.072% (-) group was 129 mg/kg/day, the average for the 0.072% (+) group was 124 mg/kg/day, the average for the 0.15% (+) group was 269 mg/kg body weight/day, and the average for the GlcN (+) group was 434 mg/kg/day (FIG. 5B).

### 2. Skin moisture content

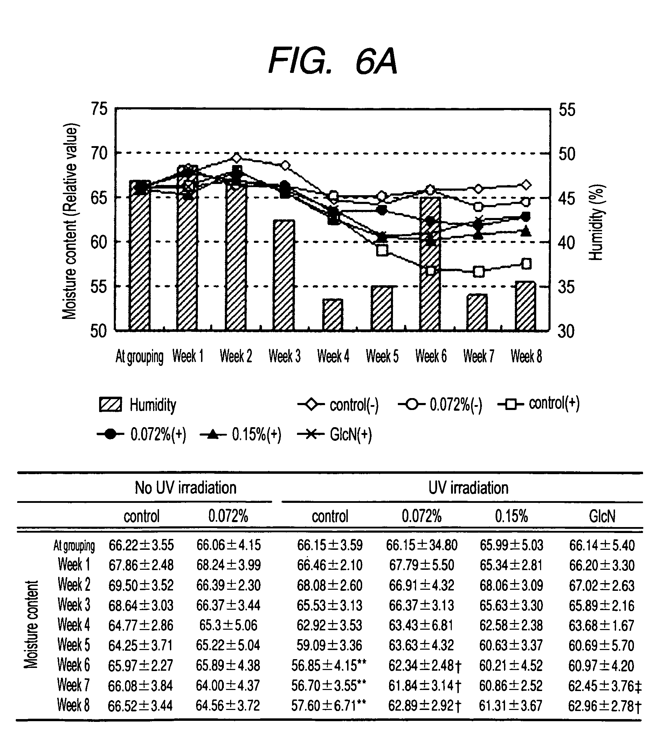

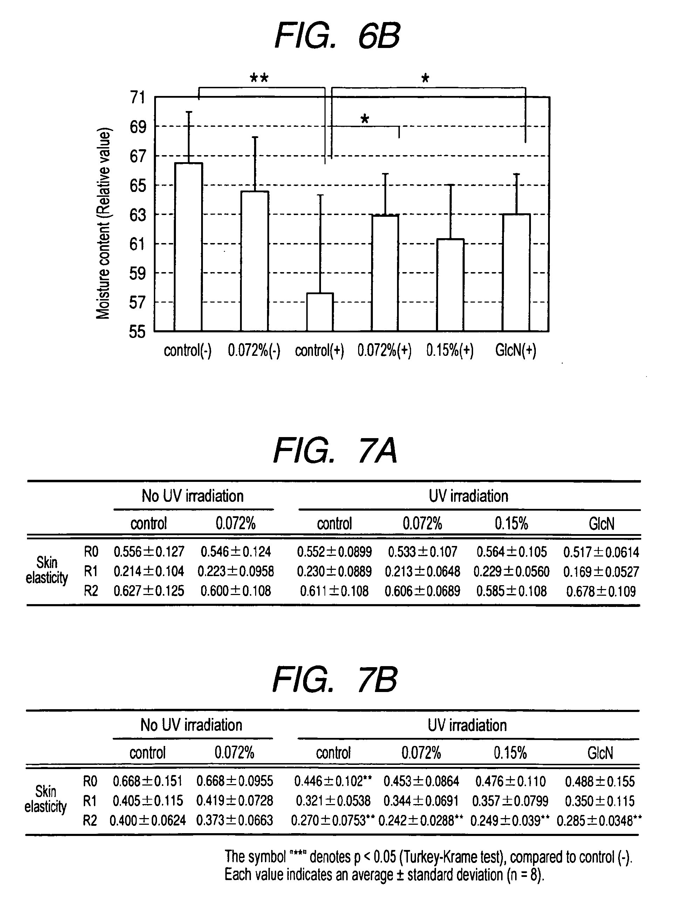

In order to investigate effects on skin moisture content by UVB irradiation and administration of a mangosteen pericarp extract, the skin moisture content was determined using CORNEOMETER CM 825. FIG. 6 shows the results. FIG. 6A shows changes over time in the moisture content and the humidity at measurements. FIG. 6B shows the moisture content at week 8.

The UVB-irradiated groups started exhibiting tendency to decrease the moisture content from around week 3. As to the skin moisture content, there was no significant difference among the respective groups up to week 5. However, from week 6 to week 8, a significant decrease in control (+) was continuously demonstrated compared to control (-). Also, a significant increase in 0.072% (+) was demonstrated compared to control (+) (p < 0.05). In addition to the above, in weeks 7 and 8, a significant increase in GlcN (+) was seen compared to control (+). As to the skin moisture content at week 8, the control (-) group was 66.52 ± 3.44, the control (+) group was 57.60 ± 6.71, the 0.072%(+) group was 62.89 ± 2.92, and the GlcN (+) group was 62.96 ± 2.78.

### 3. Skin elasticity

Skin elasticity was measured using CUTOMETER SEM575 twice at the time of grouping and the end of the study.

The measurements were conducted in triplicate for one individual, and the average of them was used as a measured value. As to R0 representing stretch of the skin, a significant decrease in control (+) was demonstrated compared to control (-). The result was obtained which indicated a decrease in stretch of the skin due to UV irradiation. In addition, as to R1 representing reformation of the skin, a significant difference was not recognized, and each group exhibited no changes. As to the R2 value representing total elasticity of the skin as reflected by these data, a significant decrease in control (+) was seen compared to control (-) (FIG. 7).

### 4. Pathological analysis

In order to investigate effects on crease formation, conditions of dorsal skin creases of mice were observed. After gas anesthesia using isoflurane, pictures were taken using a digital camera. Selected photographs of the dorsal region were shown in FIG. 8. In the control (-) group, a streak which ran in parallel against the vertebra was recognized (FIG. 8A). This streak disappeared or appeared depending on movements of the mouse. In the control (+) group, streaks which ran in a vertical orientation against the vertebra appeared (FIG. 8B). There existed an individual in which skin inflammation was verified. Alternatively, in the 0.15% (+) group, both creases ran in parallel against the vertebrate and creases ran in a vertical orientation were thin (FIG. 8C).

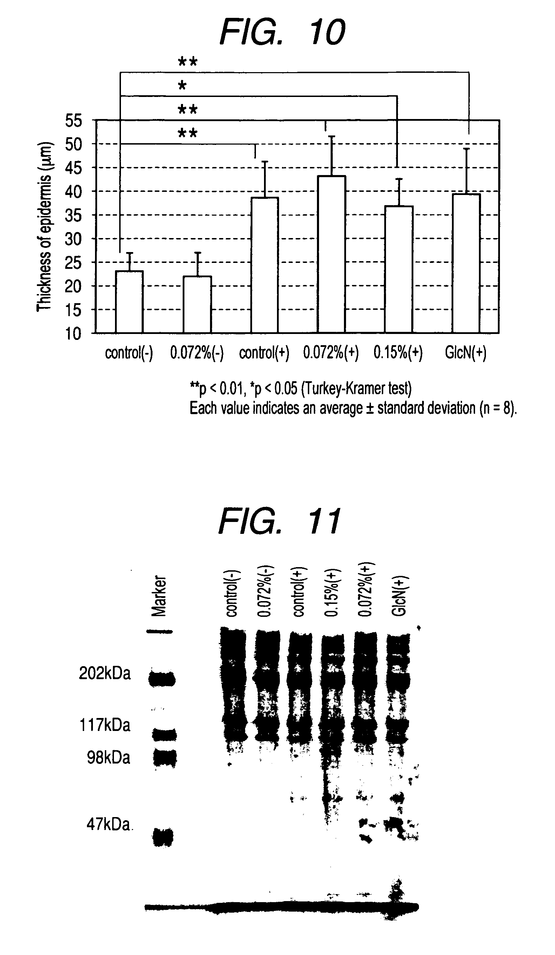

Further, HE-stained specimens were prepared, and the thickness of the epidermis in the respective groups was determined. The thickness of the epidermis was measured using the HE-stained specimens and 10 locations for each specimen were used for the measurement to obtain the average of the respective groups. FIG. 9 shows representative pictures of the HE-stained specimens for each group. FIG. 10 shows the results of measuring the thickness of the epidermis. The thickness of the epidermis of control (-) was 23.20 ± 3.56 µm, the thickness of 0.072% (-) was 21.88 ± 5.17 µm, the thickness of control (+) was 38.72 ± 7.56 µm, the thickness of 0.072% (+) was 43.17 ± 8.60 µm, the thickness of 0.15% (+) was 36.74 ± 5.92 µm, and the thickness of GlcN (+) was 39.46 ± 9.56 µm. Control (+), 0.072% (+), 0.15% (+), and GlcN (+) exhibited a significant increase in the thickness of the epidermis compared to control (-), and demonstrated hyperplasia. UV irradiation allowed for an increase in intercellular edema and intracellular edema in the basal lamina. The condition in which granule cells became sun-burn cells was verified. As to epidermic hyperplasia, the spinous layer portion, in particular, became hyperplasia. In 0.15% (+), although the hyperplasia of the spinous layer was observed, cellular conditions were similar to those of non-irradiated groups. While the intracellular edema was seen, the intercellular edema had been cured compared to 0.072% (+).

### 5. Quantification of type I collagen by SDS-PAGE and Western blotting

The skin extract of each group was used as a sample. Next, 6% polyacrylamide Tris-HCl gel was used as a separation gel, and 3% polyacrylamide Tris-HCl gel was used as a concentrating gel to thereby conduct electrophoresis, and then the gel was examined by CBB staining. Prestained SDS-PAGE Standards High Range (Control 310001920) was used as a marker. FIG. 11 shows the result. This result demonstrated that the respective samples had an equal amount of proteins.

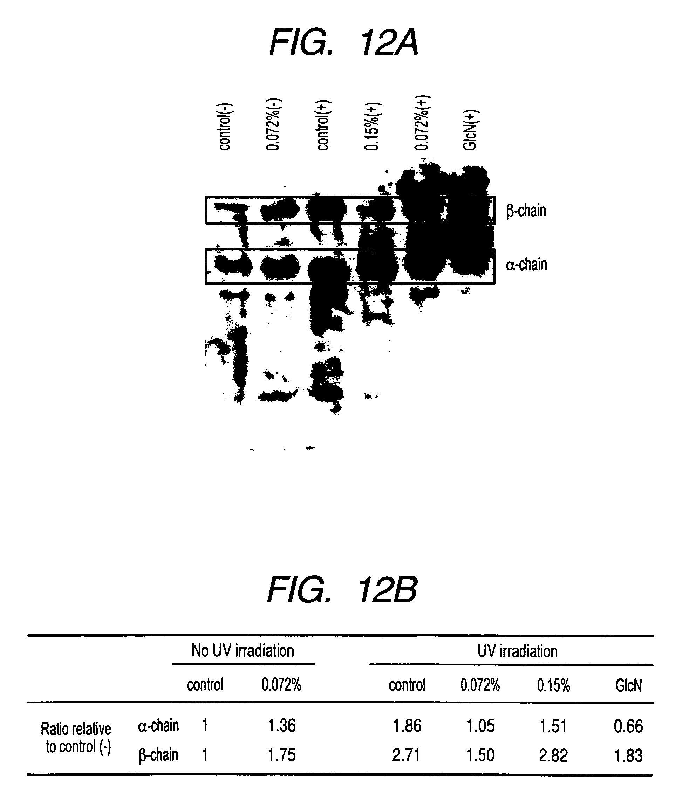

FIG. 12A shows the result of Western blotting. A band which was presumed to represent type I collagen α1-chain appeared around 117 kDa, and a band close to 200 kDa was detected which seemed to represent β-chain. FIG. 12B shows the results that intensity of bands in FIG. 12A was quantified by using an image-analyzing software, Scion Image. Regarding the band intensity, when that of control (-) was set to 1, the intensity of 0.072% (-) was 1.56, the intensity of control (+) was 2.29, the intensity of 0.15% (+) was 2.17, the intensity of 0.072% (+) was 1.28, and the intensity of GlcN (+) was 1.25 (as designated as an average between that of α-chain and that of β-chain)(FIG. 12B).

### 6. Identification of GAG by cellulose acetate membrane electrophoresis

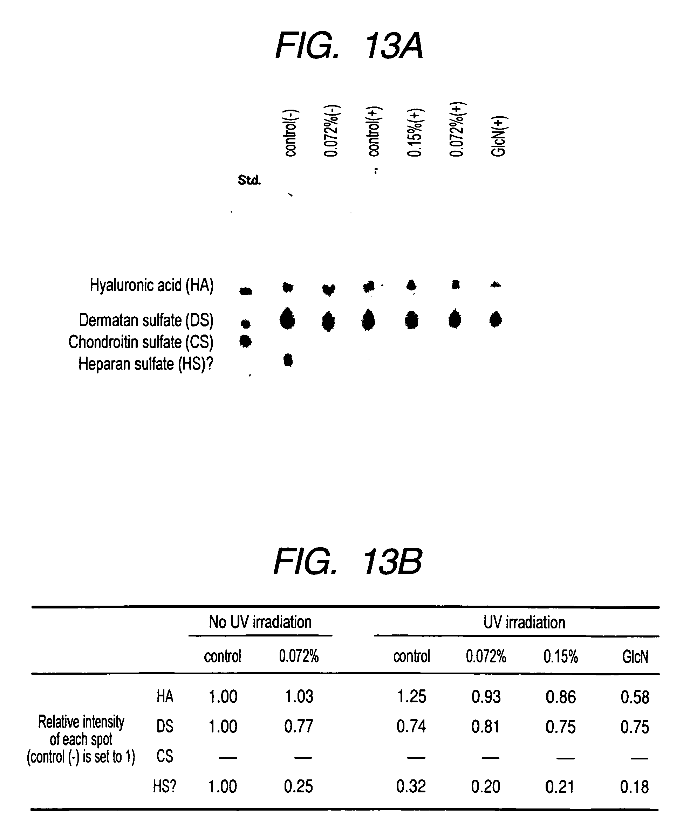

In order to identify GAG in a skin extract of a hairless mouse, and in particular an amount of hyaluronic acid (HA) which involves hydration in the skin, cellulose acetate membrane electrophoresis was carried out. FIG. 13A shows the results. FIG. 13B shows the results that the concentration of the respective spots was analyzed by an image-analyzing software, Scion Image. In respect to the band intensity of HA, when that of control (-) was set to 1, the intensity of 0.072% (-) was 1.03, the intensity of control (+) was 1.25, the intensity of 0.072% (+) was 0.93, the intensity of 0.15% (+) was 0.86, and the intensity of GlcN (+) was 0.58. Although UV irradiation increased an amount of HA, the 0.072% (+) mangosteen pericarp extract administration group which exhibited a significant increase in the skin moisture content hardly exhibited a change in the amount of HA, compared to control (+). Chondroitin sulfate (CS) was not detected. There existed a spot different from the three spots in the standard and located downstream of CS. In control (-), an intense spot was observed. This spot might be heparan sulfate (HS), however, its identification remains undone.

### 7. Quantification of lipid peroxide in the skin

In order to investigate antioxidant actions of a mangosteen pericarp extract on the skin, lipid peroxide was determined by utilizing a TBA reaction. At occurrence of oxidative stress *in vivo,* the substance most susceptible to its damage is a lipid containing polyunsaturated fatty acid. As well as lipid hydroxy peroxides, a thiobarbituric acid-reactive substance (TBARS) has been used as an oxidative stress marker *in vivo.* This method is understood as a useful method for comprehensively determining a degree of lipid peroxidation. A reaction in Ohkawa protocol can identify what kind of substance is TBARS in a sample by adding EDTA or Fe ion. Generation of red pigments derived from alkenal and alkadienals is known to be inhibited by EDTA and to be enhanced by addition of Fe ion.

In the present test, when nothing was added, there was no coloring. However, when Fe ion was added, there was coloring. Accordingly, a protocol for adding Fe ion was adopted.

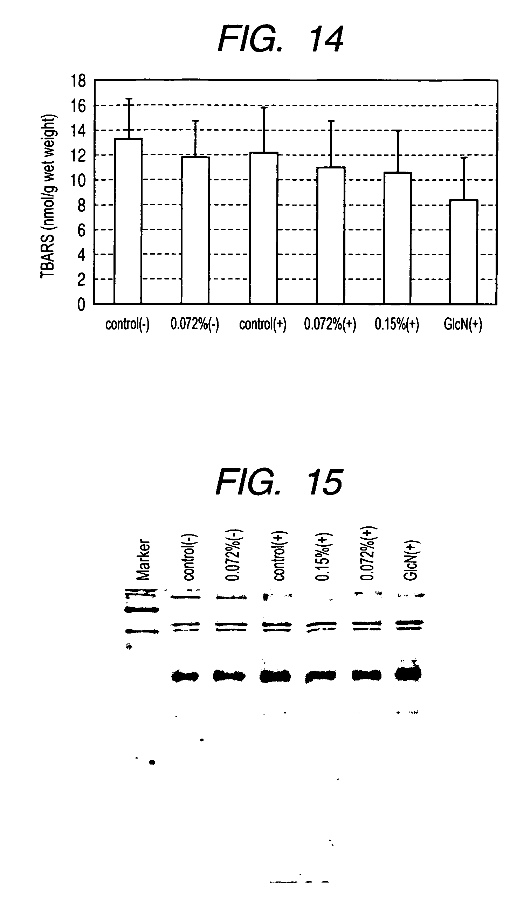

FIG. 14 shows the measured results. According to the TBARS measurement results, a significant difference among the respective groups was not detected.

### 8. Quantification of carbonyl proteins

Proteins are one of factors affected by oxidative damage. In the skin which has been subjected to photoaging, accumulation of damage caused by ROS occurs in proteins localized in the upper part of the dermis (Sander CS, 2002). Examples of the oxidative modification in proteins include a side chain structure having aldehyde or ketone (carbonyl proteins), a tyrosine cross-linking structure, an amino acid substitution, oxidation of amino acid, and cleavage of a peptide bond. Oxidative damage in proteins causes, for example, a change in an enzymatic activity, loss of functions of a structural protein, and a change in susceptibility to protein degradation (Shacter E, 2000). Among the proteins having an oxidative modification, the carbonyl proteins have been frequently quantified as a representative example.

Extracts were obtained from the skin tissue of the respective groups by using a protein-extracting solution (1% Trion X-100/50 mM Tris-HCl/75 mM NaCl/10 mM EDTA-2Na/5 mM benzamidine hydrochloride/0.1 M aminohexanoic acid). These extracts were electrophoresed as samples, and then levels of proteins in each sample were examined by using CBB staining. Here, 10% polyacrylamide Tris-HCl gel was used as a separation gel, and 3% polyacrylamide Tris-HCl gel was used as a concentrating gel. Prestained SDS-PAGE Standards High Range (Lot. No. Control 310001920) was used as a marker. FIG. 15 shows the results. This result demonstrated that the respective samples had an equal amount of proteins.

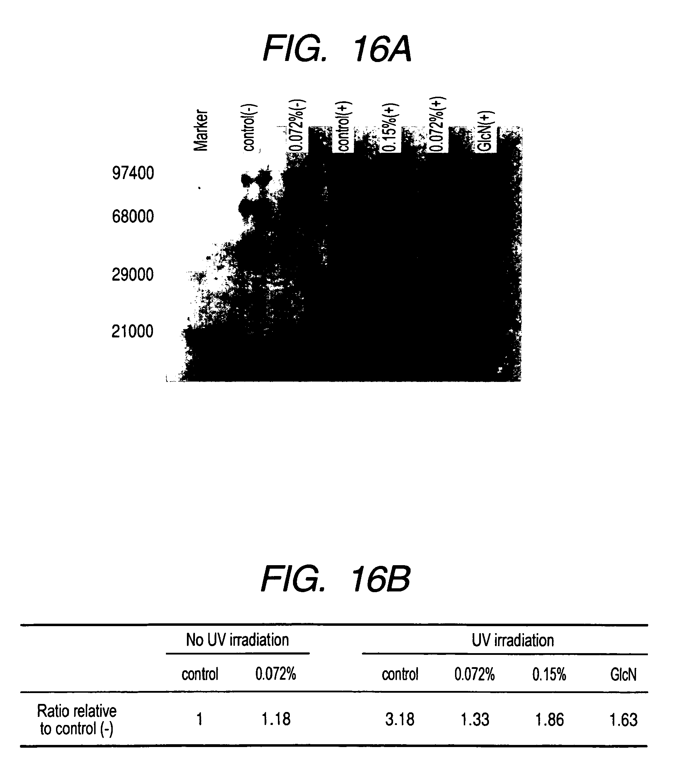

Evaluation of carbonyl proteins in each sample was carried out according to a "Method for determining oxidized proteins in a skin tissue" as described above. FIG. 16A shows the results of Western blotting of each sample as obtained in this evaluation method. A rabbit anti-DNP antibody was used as a primary antibody, and a goat anti-rabbit IgG (HRP-conjugated) was used as a secondary antibody. Intense bands around molecular weights 97.4 kDa and 68 kDa were detected. In addition, the band intensity was quantified in terms of density by using an image-analyzing software, Scion Image. The intensity of control (-) was set to 1, and relative intensity was indicated (FIG. 16B). When the intensity of control (-) was set to 1, the intensity of 0.072% (-) was 1.18, the intensity of control (+) was 3.18, the intensity of 0.072% (+) was 1.33, the intensity of 0.15% (+) was 1.86, and the intensity of GlcN (+) was 1.63. The band intensity of 0.072% (+) was about 42% of that of control (+).

### 9. Quantification of lipid peroxide in plasma

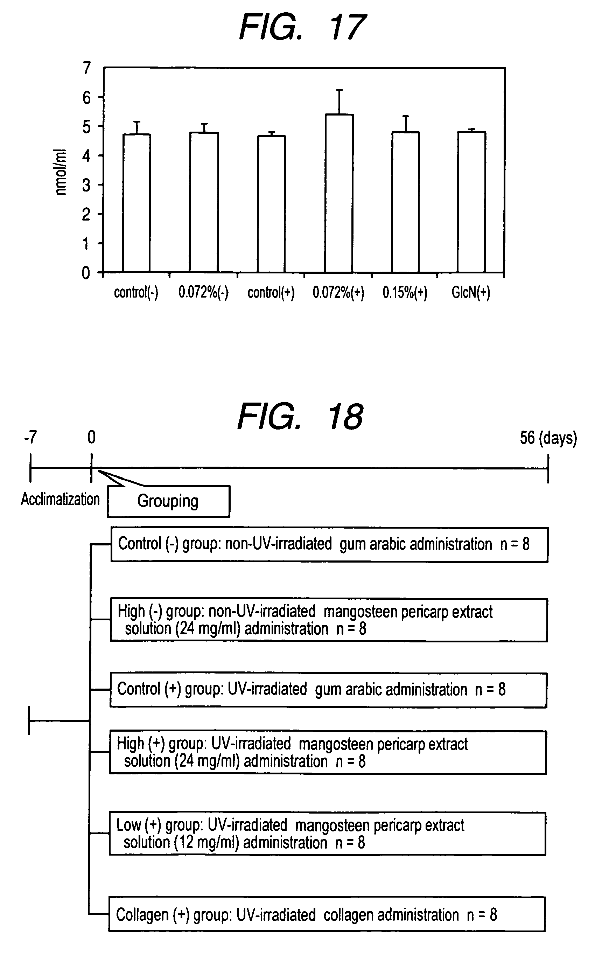

The concentration of lipid peroxide in plasma was measured. However, a significant difference was not recognized (FIG. 17).

### Discussion

In view of the above, UVB irradiation decreased skin moisture content and skin elasticity in a control group, but administration of a mangosteen pericarp extract was verified to increase the skin moisture content.

A mangosteen is known to contain 130 kinds or more of active ingredients. In the present invention, a plurality of components seem to exert an antioxidant action by comprehensive and synergetic action thereof, and further synergistically exert effects other than the antioxidant action, which remarkably achieves treatment and/or prevention of skin disorder.

The measurement range of Corneometer is between 30 and 40 µm. When used in a hairless mouse, this apparatus seems to measure moisture content of a part of the stratum corneum and the epidermis. According to the results of the pathological analysis, it has been found that UV irradiation causes hyperplasia in the epidermis, in particular a spinous layer, and that intracellular edema in a basal cell and a prickle cell and intercellular edema appear. Treatment effects on the edema conditions was observed in the 0.15% mangosteen pericarp extract administration group compared to the 0.072% mangosteen pericarp extract administration group. Also, the 0.15% (+) group exhibited a lower degree of hyperplasia. From this observation, it was suggested that oral ingestion of a mangosteen pericarp extract had a therapeutic effect on pathological changes in the epidermis, and it was indicated that the effect thereof was more remarkable in the 0.15% mixed-diet group. However, the 0.072% mixed-diet administration group exhibited an increase in the moisture content.

As a factor involving an increase in the skin moisture content, hyaluronic acid (HA) and collagen in the skin were quantified. In the quantification of type I collagen, about 2.1 fold increase in the band intensity was obtained in a UV-irradiated control group, compared to a non-UV-irradiated control group. This seems to be caused by an increase in collagen because, in an initial stage of UV irradiation, an *in vivo* protection mechanism functions and the production tends to increase. Administration of a mangosteen pericarp extract decreased production of collagen. This seems to be because an amount of collagen does not tend to increase due to reduction of the UV irradiation damage.

In view of the results of determining a marker of an oxidative stress by detecting carbonyl proteins, the results were obtained that the oxidative stress increased by UV irradiation and that ingestion of a mangosteen pericarp extract decreased the stress. The antioxidant action of the mangosteen pericarp extract was suggested to be deeply involved with treatment of the skin conditions.

The above demonstrated that, in the experiments involving a mixed-diet administration, oral ingestion of a mangosteen pericarp extract exhibited an increase in the moisture content. From the results of detecting carbonyl proteins that constitute one of markers for oxidative stress, it is suggested that an inhibition of the oxidative stress is involved with the moisture content increase which is caused by the administration of the mangosteen pericarp extract.

### Example 3

### Verification of effects of improving skin disorder by oral forced administration of a mangosteen pericarp extract 1. Preparation of administration samples and oral administration

An administration sample employed a mangosteen pericarp formulation. Specifically, a formulation having a ratio of a mangosteen pericarp extract:gum arabic = 1:1 was used. One having only gum arabic was used as a control, and collagen was used as a positive control. Solutions having respective concentrations were prepared. A solution having a concentration of 0.1 ml/10 g body weight was orally administered every day during the rearing period by using a stomach tube. Two kinds (a high concentration (24 mg/ml) and a low concentration (12 mg/ml)) of a solution containing a mangosteen pericarp extract were prepared. Then, 24 mg/ml of a gum arabic solution and 20 mg/ml of a collagen solution were prepared.

### 2. Rearing schedule

FIG. 18 shows a study schedule. After 1 week of acclimatization, grouping was conducted based on skin moisture content and viscoelasticity. Animals were divided into six groups including a non-UV-irradiated gum arabic administration group (control (-) group), a non-UV-irradiated mangosteen pericarp extract-solution (24 mg/ml) administration group (high (-) group), a UV-irradiated gum arabic administration group (control (+) group), a UV-irradiated mangosteen pericarp extract-solution (24 mg/ml) administration group (high (+) group), a UV-irradiated mangosteen pericarp extract-solution (12 mg/ml) administration group (low (+) group), and a UV-irradiated collagen administration group (collagen (+) group). UVB irradiation was carried out three times a week, and forced oral administration was daily conducted by using a tube. The study period was set to 8 weeks, and the study was carried out from May to July in 2007.

### 3. As to UVB irradiation

UVB irradiation was conducted three times a week using UVB lump GL20SE (SANKYO DENKI). The irradiation intensity was 0.3 mW/cm². Irradiation intensity was adjusted by using a digital ultraviolet light intensity meter, UV-340 (AS ONE Corporation). Mice were left for one and a half hour in an individualized cage having a size of 9 cm × 5 cm × 4 cm. In order to decrease a difference in irradiation intensity for respective cages, the mice were irradiated while subjected to a rotation in every occasion.

The irradiation period for week 1 was 1 minute, the period for week 2 increased to 2 minutes, and the period for week 3 increased to 3 minutes. After that, the irradiation was conducted for 3 minutes. After day 38, the irradiation intensity was made to increase to 4 minutes. However, since erythema appeared on the skin in the mice, the irradiation was conducted for 3 minutes and 30 seconds after day 43. The total irradiation amount was 1.224 J.

### 4. Method for extracting proteins from a skin tissue

A skin tissue as sampled in a manner similar to Example 2 was weighed, collected for the respective groups, and degreased with ethanol for 24 hours. After divided into thin stripes by using scissors, the skin tissue was washed twice with protease inhibitor-containing PBS (-) (PBS (-) in which 5 mM benzamidine hydrochloride, 10 mM EDTA-2Na, and 0.1 M aminohexanoic acid were dissolved) to remove serum components, etc. Then, 10 volumes per skin wet weight of a protein-extracting solution (4 M guanidinium hydrochloride/50 mM Tris-HCl/0.1 M NaCl/5 mM benzamidine hydrochloride/10 mM EDTA-2Na/0.1 M aminohexanoic acid (pH 7.4)) were added, and the mixture was shaken using a shaker at 4°C for 72 hours. The mixture was centrifuged at 4000 rpm for 20 minutes, and the supernatant was collected. Then, dialysis was carried out by using a buffer (pH 7.4) containing 7 M urea/50 mM Tris-HCl/0.1 M NaCl/5 mM benzamidine hydrochloride/10 mM EDTA-2Na/0.1 M aminohexanoic acid. After the dialysis, TOYOPEARLⓇ DEAE-650S (manufactured by Tosoh Corporation) were added, and the mixture was stirred at 4°C for 24 hours to separate into an absorbed fraction (A) and an unabsorbed fraction (B).

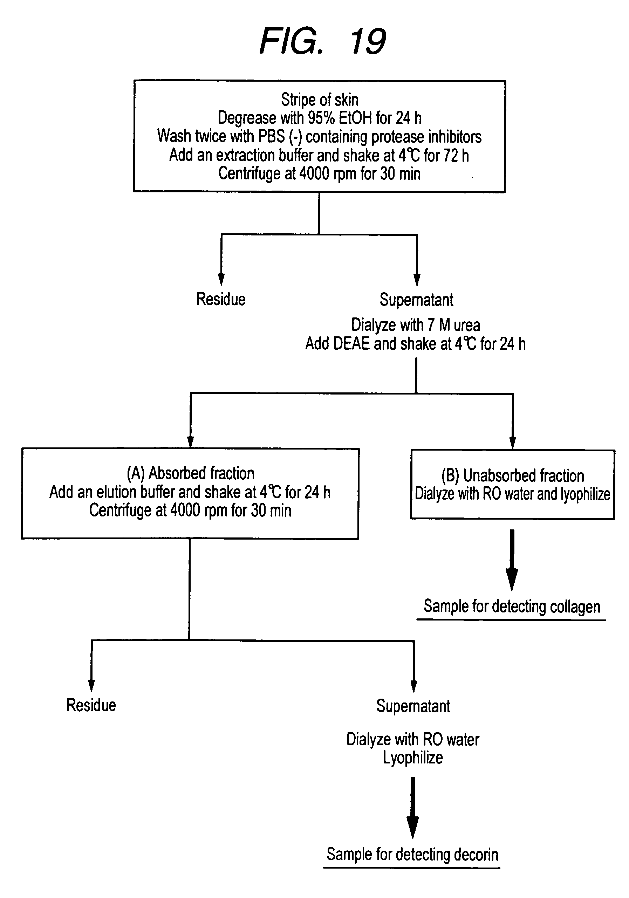

To fraction A was added an elution buffer (7 M Urea/50 mM Tris-HCl/2 M NaCl/5 mM benzamidine hydrochloride/10 mM EDTA-2Na/0.1 M aminohexanoic acid (pH 7.4)). The mixture was extracted at 4°C for 72 hours while stirring, and dialysis was then conducted against RO water. As to fraction B, dialysis was conducted against RO water as it was. Both fractions A and B were subjected to lyophilization after the dialysis, and were used as samples for SDS-PAGE and Western blotting. Fraction A was used for detecting decorin, and fraction B was used for detecting collagen. FIG. 19 illustrates these steps. 5. The other experiments were carried out in a manner similar to Example 2.

### Results

### 1. Skin moisture content

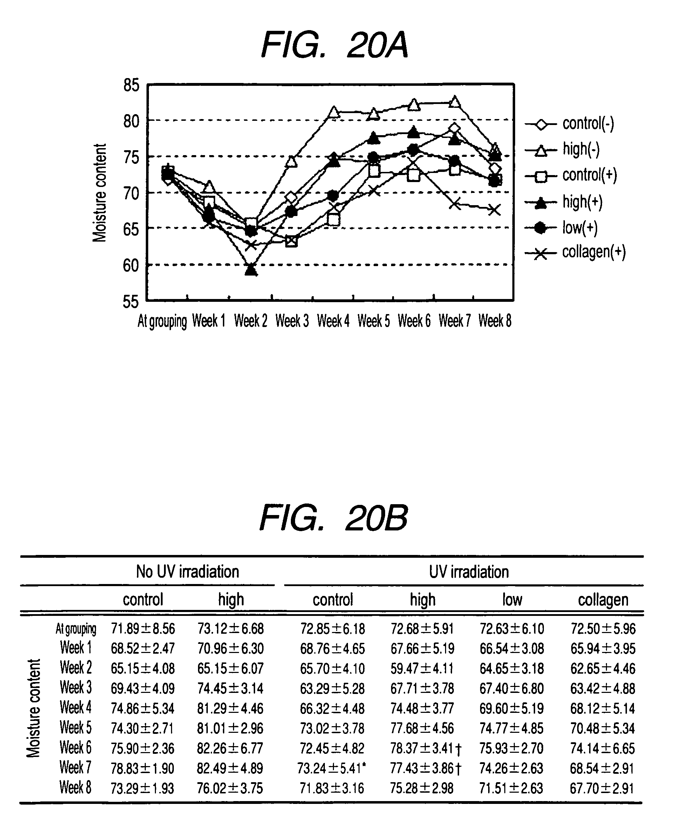

In order to investigate effects of UVB irradiation and administration of a mangosteen pericarp extract on skin moisture content, the skin moisture content was determined using CORNEOMETER CM 825. FIGS. 20A and 20B show the results. A difference among the respective groups was not detected up to week 5. At week 6, control (+) exhibited tendency to decrease moisture content compared to control (-) (p < 0.1). In addition, a significant increase was observed for high (+) compared to control (+). At week 7, a significant decrease (p < 0.05) was detected in control (+) compared to control (-), and a significant increase was detected in high (+) compared to control (+). The skin moisture content at week 7, in which a significant difference was seen, of control (-) was 78.83 ± 1.90, the content of high (-) was 82.49 ± 4.89, the content of control (+) was 73.24 ± 5.41, the content of high (+) was 77.43 ± 3.86, the content of low (+) was 74.26 ± 2.63, and the content of collagen (+) was 68.54 ± 2.91.

### 2. Skin elasticity

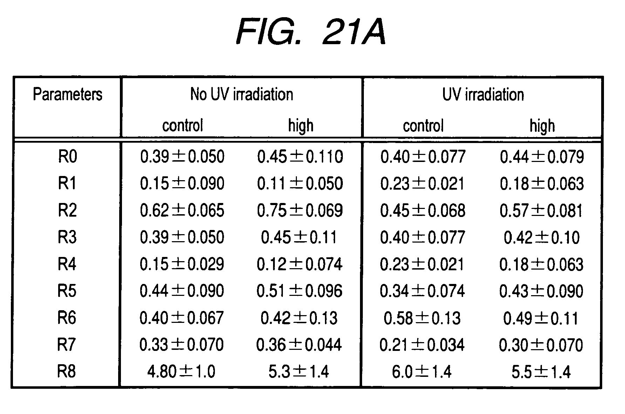

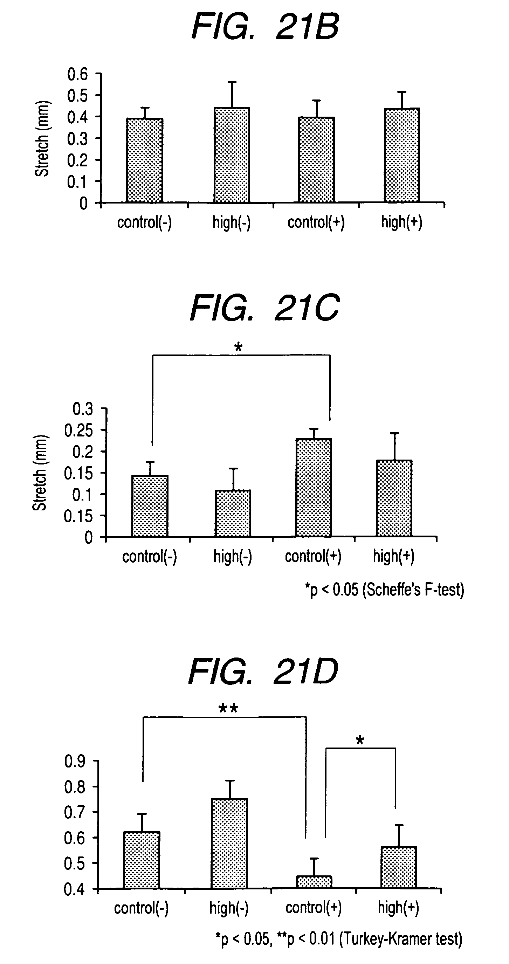

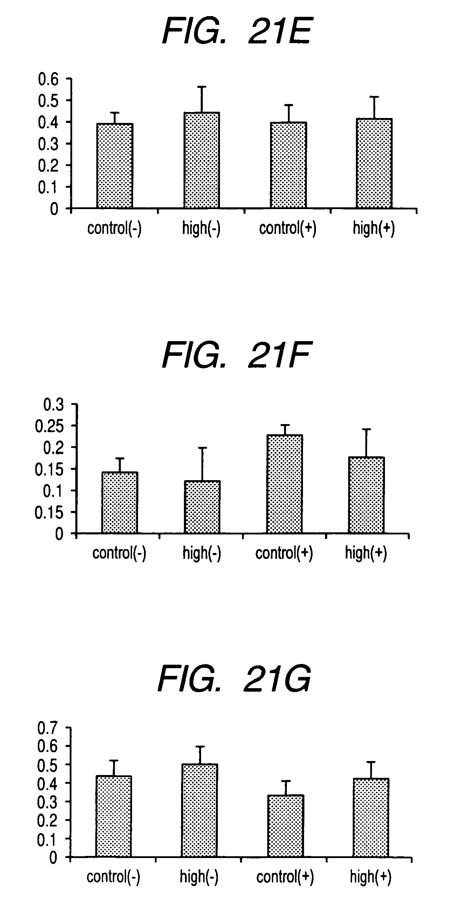

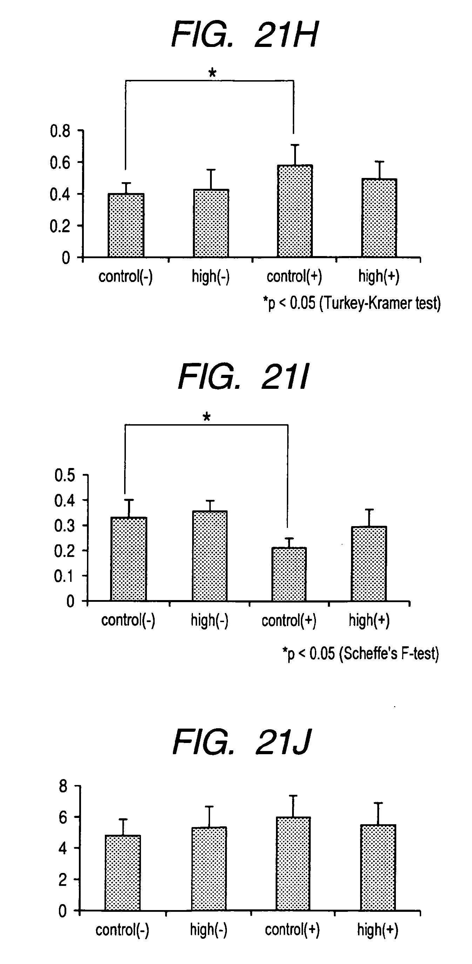

Elasticity of the skin of a lumbar region in hairless mice was determined using CUTOMETER SEM575. FIGS. 21A to J show the results. At the grouping, a significant difference was not detected. At the dissection, a significant difference was not observed among the respective groups in R0 which is a parameter indicating stretch of the skin. However, as to R1 indicating reformation of the skin, a significant decrease (p < 0.05) was demonstrated in control (+) compared to control (-). In addition, as to R2 indicating total elasticity of the skin, a significant decrease (p < 0.01) was demonstrated in control (+) compared to control (-), and a significant increase (p < 0.05) was demonstrated in high (+) compared to control (+). Also, a significant decrease was verified in high (+) compared to high (-).

### 3. Pathological analysis

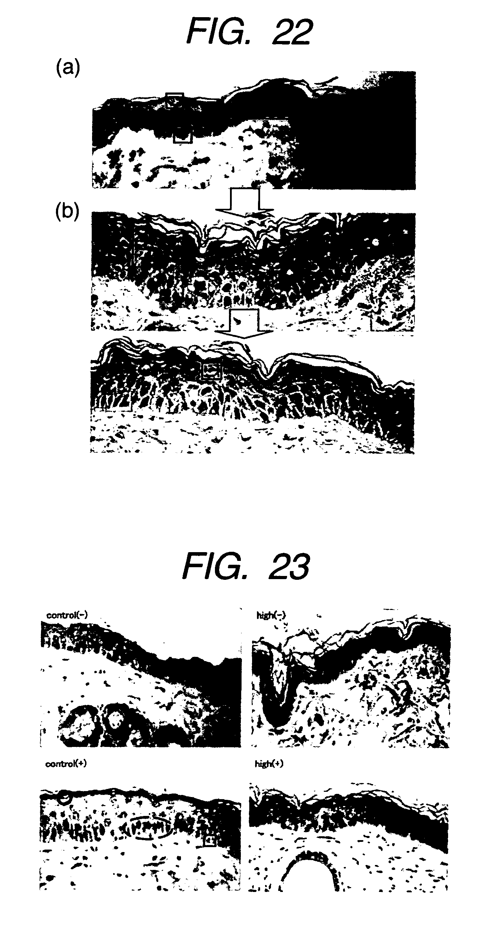

FIG. 22 illustrates notable findings on the HE-stained specimens of the dorsal skin. FIG. 22 shows that intracellular edema in a basal cell layer is rarely observed in non-UV-irradiated specimens (a), but the intracellular edema in a basal cell layer is observed in irradiated specimens (b). It is demonstrated that reception of further damage causes appearance of intercellular edema and causes the edema to spread from a basal cell layer to a prickle cell layer. In addition, cells which have become sun-burn cells are identified in granule cells. Specifically, the following three points have been focused: in the HE-stained specimens, (1) whether or not there are cells in which granule cells have become sun-burn cells; (2) whether or not there are intracellular edema in basal cells and intercellular edema; and (3) whether or not the intercellular edema spreads over a spinous layer. As to the edema, when the damage was small, only intracellular edema was observed. However, as the damage became large, occurrence of intercellular edema increased. Then, the intercellular edema in a basal lamina spread over a spinous layer.

FIG. 23 shows the results of the pathological analysis of the respective groups. FIG. 23 shows representative specimens of each group. In control (-), occasional intracellular edema was able to be observed. In UV-irradiated groups, in addition to intracellular edema, intercellular edema spread. In some of specimens, the intercellular edema spread over a spinous layer. Conditions of edema apparently improved in high (+), compared to control (+). In addition, in FIG. 23, an area surrounded by a double line indicates a pathological finding that a granule cell has become a sun-burn cell. An area surrounded by a single line indicates a pathological finding of intracellular edema of a basal cell. An area surrounded by a dashed line indicates a pathological finding of intercellular edema of a basal cell.

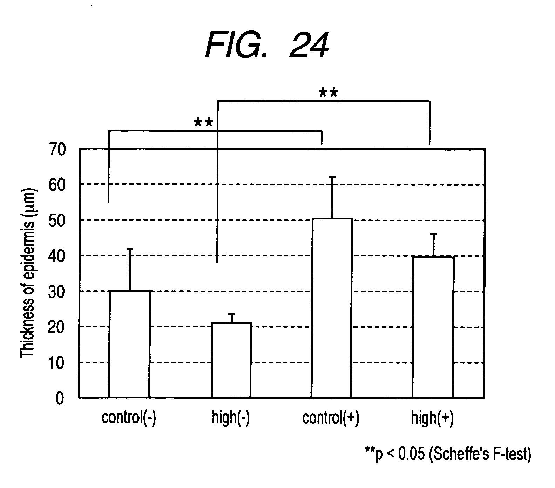

Also, FIG. 24 shows the results of measuring the thickness of the epidermis. The thickness of the epidermis was determined for 10 locations of the respective specimens, and the average was calculated to yield these values. The thickness of the epidermis of control (-) was 29.87 ± 11.94 µm, the thickness of high (-) was 21.03 ± 2.46 µm, the thickness of control (+) was 50.42 ± 11.89 µm, and the thickness of high (+) was 39.63 ± 6.63 µm. Epidermic hyperplasia was observed in control (+) compared to control (-) and in high (+) compared to high (-) (FIG. 24).

In addition, when conditions of creases in the dorsal skin were observed, it was demonstrated that deep creases formed in control (+) compared to control (-) group. A similar situation was observed when high (-) group and high (+) group were compared. When control (-) group and high (-) group were visually compared, a difference was unable to be observed by a visual inspection. When control (+) group and high (+) group were compared, a certain difference could be felt. However, a difference that indicated an apparent difference was unable to be observed.

### 4. SDS-PAGE and Western blotting of type I collagen

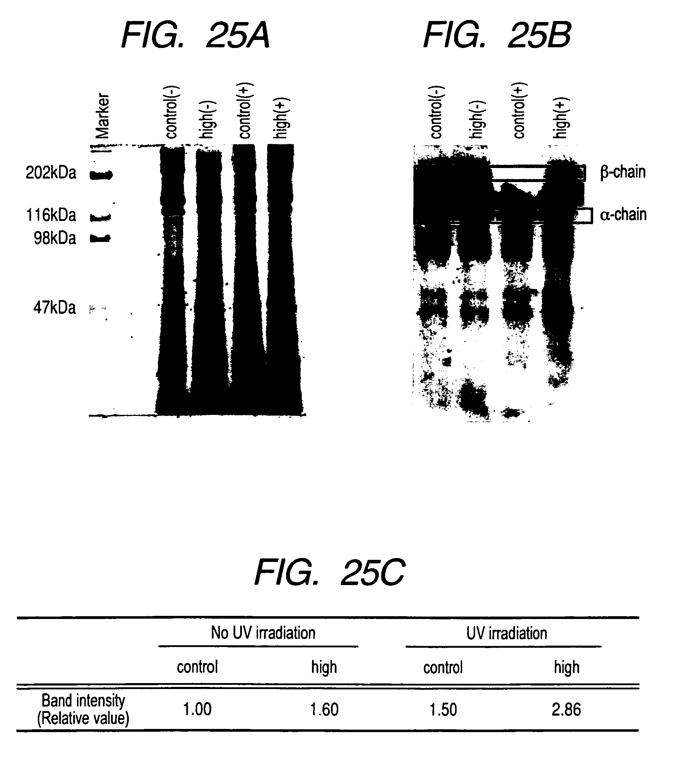

6% polyacrylamide gel was used as a separation gel, and 3% polyacrylamide gel was used as a concentrating gel. Prestained SDS-PAGE Standards High Range (Control 310001920) was used as a marker to conduct electrophoresis. Then the CBB staining demonstrated that the respective samples had an equal amount of proteins. FIGS. 25A and B show the results of Western blotting. A band which seemed to represent type I collagen α-chain was identified around 116 kDa. When the band intensity of control (-) was set to 1, the intensity of high (-) was 1.60, the intensity of control (+) was 1.50, and the intensity of high (+) was 2.86 (FIG. 25C).

### 5. SDS-PAGE and Western blotting of decorin

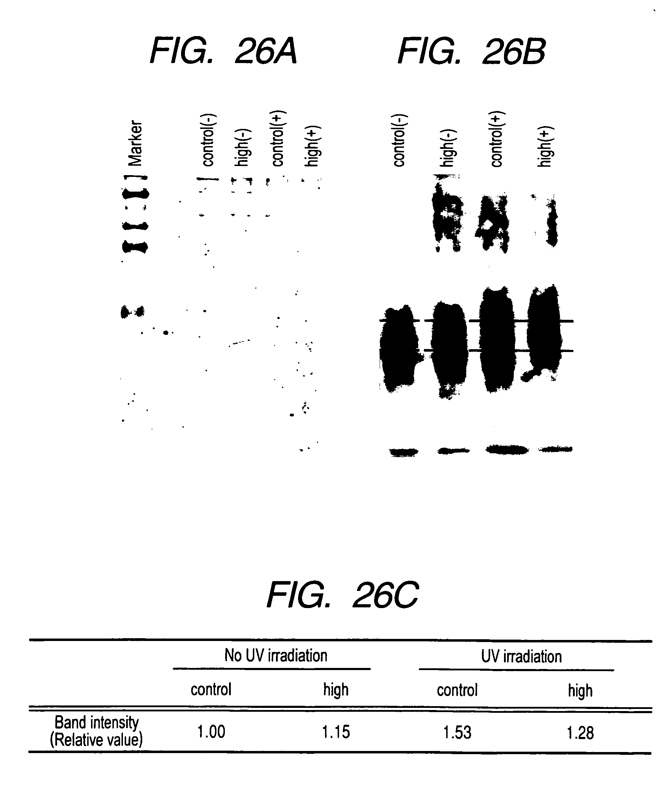

First, 7.5% polyacrylamide gel was used as a separation gel, and 3% polyacrylamide gel was used as a concentrating gel. Prestained SDS-PAGE Standards High Range (Lot. No. Control 310001920) was used as a marker to conduct SDS-PAGE. Then the CBB staining demonstrated that the respective samples had an equal amount of proteins. After that, Western blotting was carried out (FIGS. 26A and B). When the band intensity of control (-) was set to 1, the intensity of high (-) was 1.15, the intensity of control (+) was 1.53, and the intensity of high (+) was 1.28 (FIG. 26C). In the UV-irradiated control group, bands of decorin spread to both a lower-molecular-weight side and a higher-molecular-weight side compared to the non-UV-irradiated control group. In high (+) group, bands located at the lower-molecular-weight side disappeared.

### Discussion

In Examples of the present invention, the results have been achieved that administration of a mangosteen pericarp extract improves skin moisture content which has been decreased by UV irradiation in a hairless mouse. Thus, the mangosteen pericarp extract is considered to be a substance effective in skin hydration.

In addition, as a dispersant for a tube sample, gum arabic which seemed to have no effects of improving the moisture content was used. However, it was observed that the moisture content tended to increase in a control group. There is a report (International Journal of Pharmaceutics 298: 153-263, 2005) that in a simplified *in vitro* model of the stratum corneum, gum arabic inhibits lipid peroxidation. This report suggests that gum arabic may be indicated to function to inhibit oxidative stress that is generated by UVB irradiation.

Also, as to skin elasticity, it was suggested that UV irradiation decreased total elasticity of the skin, but a mangosteen pericarp extract had a recovering effect on elasticity that had been decreased.

The results of Western blotting indicated that the band intensity of collagen increased by UV irradiation. There is a report that the total collagen amount in the skin does not necessarily decrease by ultraviolet irradiation. This result has an agreement with that report. Regardless of no irradiation and/or irradiation, administration of the pericarp extract is found to recover pathological findings such as edema to begin with. Thus, the administration is presumed to have some sorts of mechanisms of recovering the skin tissue, which results in an increase in collagen production.

From the results of Western blotting of decorin, the results were obtained that administration of the pericarp extract decreased low-molecular-weight and high-molecular weight decorin which had been generated by UV irradiation. Decorin is a proteoglycan having a molecular weight of about 100 kDa. One chondroitin sulfate chain or dermatan sulfate chain covalently binds to a core protein having about 40 kDa. The core protein is a proteoglycan having a relatively low molecular weight and having a leucine-rich repeat (LRR) sequence including 8 to 10 amino acids. Decorin interacts with collagen or another molecule, and plays a key role in formation for and functions in a connective tissue. It has been reported that decorin-gene-deficient mice have weakened skin and loss of elasticity, compared to a wild-type counterpart. In addition, as to regulation of fiber formation, there is a report that topical administration of decorin to injured muscle fibers inhibits excessive fiber formation and improves muscle repair. These reports indicate that decorin involves normal construction of extracellular matrix. UV irradiation increases a lower-molecular-weight and higher-molecular-weight decorin than normal conditions. The decorin is primarily associated with a decrease in skin elasticity between moisture content and the elasticity. Administration of the pericarp extract allows for disappearance of the lower-molecular-weight bands of decorin and an increase in the elasticity. Accordingly, the increase in the lower-molecular-weight decorin is considered to have a decreasing action on the skin elasticity. Also, the administration of the pericarp extract is considered to function in inhibiting production of the lower-molecular-weight decorin. Detailed mechanisms require further investigations.

As described above, administration of a mangosteen pericarp extract recovers skin moisture content, skin elasticity, and hyperplasia of the epidermis. In addition, aggravation of pathological findings caused by UV irradiation is made to improve. Among them, inhibition of abnormal decorin production due to UV irradiation is presumed to have a relationship with recovery of the skin elasticity.

### Example 4

### Preparation of food using a mangosteen extract

A chewing gum was prepared using a mangosteen pericarp extract as prepared in Example 1 as follows:

| | |
|---|---|
| Gum base | 20.0% |
| Sugar | 54.7 |
| Glucose | 14.5 |
| Starch syrup | 9.3 |
| Flavoring agent | 0.5 |
| Mangosteen pericarp extract | 1.0 |
| | 100.0%. |

A candy was prepared using a mangosteen pericarp extract as prepared in Example 1 as follows:

| | |
|---|---|
| Sugar | 50.0% |
| Starch syrup | 33.4 |
| Citric acid | 1.0 |
| Flavoring agent | 0.2 |
| Mangosteen pericarp extract | 1.0 |
| Water | 14.4 |
| | 100.0%. |

A gummy jelly was prepared using a mangosteen pericarp extract as prepared in Example 1 as follows:

| | |
|---|---|
| Gelatin | 60.0% |
| Starch syrup | 23.0 |
| Sugar | 7.5 |
| Vegetable oil and fat | 4.5 |
| Mannitol | 3.0 |
| Lemon juice | 1.0 |
| Mangosteen pericarp extract | 1.0 |
| | 100.0%. |

A chocolate was prepared using a mangosteen pericarp extract as prepared in Example 1 as follows:

| | |
|---|---|
| Sugar powder | 40.8% |
| Cacao bitter | 20.0 |
| Whole milk powder | 20.0 |
| Cacao butter | 17.0 |
| Mannitol | 1.0 |
| Mangosteen pericarp extract | 1.0 |
| Flavoring agent | 0.2 |
| | 100.0%. |

A sorbet was prepared using a mangosteen pericarp extract as prepared in Example 1 as follows:

| | |
|---|---|
| Orange juice | 25.0% |
| Sugar | 25.0 |
| Egg white | 10.0 |
| Mangosteen pericarp extract | 0.1 |
| Flavoring agent | 0.1 |
| Water | 39.8 |
| | 100.0%. |

An ice cream was prepared using a mangosteen pericarp extract as prepared in Example 1 as follows:

| | |
|---|---|
| Degreased milk powder | 50.0% |
| Whipped cream | 25.0 |
| Sugar | 10.0 |
| Egg yolk | 10.0 |
| Mangosteen pericarp extract | 1.0 |
| Flavoring agent | 0.1 |
| Water | 3.9 |
| | 100.0%. |

A biscuit was prepared using a mangosteen pericarp extract as prepared in Example 1 as follows:

| | |
|---|---|
| First-grade soft wheat flour | 25.0% |
| First-grade medium-strength flour | 22.0 |
| Refined sugar | 5.0 |
| Sodium chloride | 1.0 |
| Glucose | 1.0 |
| Palm shortening | 12.0 |
| Sodium bicarbonate | 0.2 |
| Sodium bisulfite | 0.2 |
| Rice powder | 2.0 |
| Whole milk powder | 1.0 |
| Milk powder substitute | 0.6 |
| Mangosteen pericarp extract | 1.0 |
| Water | 29.0 |
| | 100.0%. |

A fruit tablet was prepared using a mangosteen pericarp extract as prepared in Example 1 as follows:

| | |
|---|---|
| Sugar | 75.8% |
| Glucose | 19.0 |
| Sucrose fatty acid ester | 0.2 |
| Flavoring agent | 0.2 |
| Mangosteen pericarp extract | 0.8 |
| Water | 4.0 |
| | 100.0%. |

A beverage was prepared using a mangosteen pericarp extract as prepared in Example 1 as follows:

| | |
|---|---|
| Orange juice | 30.0 % |
| Isomerized sugar | 15.14 |
| Citric acid | 0.1 |
| Vitamin C | 0.04 |
| Flavoring agent | 0.1 |
| Mangosteen pericarp extract | 0.2 |
| Water | 54.42 |
| | 100.0 %. |

The present application claims benefit of Japanese Patent Application No. 2009-085411, filed on March 31, 2009, which is hereby incorporated by reference herein in its entirety.

## Claims

1. A composition for treatment and/or prevention of skin disorder, comprising an extract extracted from a mangosteen (*Garcinia mangostana L.*) pericarp by using a polar solvent.

2. The composition for treatment and/or prevention of skin disorder according to claim 1, wherein the polar solvent is ethanol or an ethanol aqueous solution.

3. The composition for treatment and/or prevention of skin disorder according to claim 1 or 2, wherein the skin disorder is caused by reactive oxygen.

4. The composition for treatment and/or prevention of skin disorder according to any one of claims 1 to 3, wherein the skin disorder is caused by ultraviolet irradiation.

5. The composition for treatment and/or prevention of skin disorder according to any one of claims 1 to 4, wherein a decrease in skin moisture content due to ultraviolet irradiation is reduced.

6. The composition for treatment and/or prevention of skin disorder according to any one of claims 1 to 5, wherein skin injury caused by ultraviolet irradiation is reduced.

7. A food comprising a composition for treatment and/or prevention of skin disorder according to any one of claims 1 to 6.
